# EUROPÄISCHE PATENTSCHRIFT

(11) **EP 1 250 909 B1**
(45) Veröffentlichungstag und Bekanntmachung des Hinweises auf die Patenterteilung: **06.10.2004**
(21) Anmeldenummer: 02008216.0
(22) Anmeldetag: 18.04.2002
(51) Int. Cl.: A61K 7/13

(54) **Haarfärbemittel**
Hair dye composition
Teinture pour cheveux

(30) Priorität: 18.04.2001 DE 10118893
(43) Veröffentlichungstag der Anmeldung: 23.10.2002
(73) Patentinhaber: KPSS-Kao Professional Salon Services GmbH, 64297 Darmstadt (DE)
(72) Erfinder: Lorenz, Heribert, 64401 Gross-Bieberau (DE)

(56) Entgegenhaltungen:
- WO-A-96/09807
- FR-A- 1 542 505
- FR-A- 2 746 312

## Beschreibung

Die vorliegende Erfindung betrifft ein Haarfärbemittel auf Basis eines mit Peroxid reagierenden Oxidationsfarbstoff-Systems, das dauerhafte intensive Farbtöne liefert, die entweder als solche angewandt werden, oder, in Kombination mit weiteren Entwickler- und/oder Kupplersubstanzen, zur Erzielung weiterer Farbnuancen benutzt werden können.

Die nach wie vor in Haarfärbemitteln meist eingesetzten Entwicklersubstanzen sind 1,4-Diaminobenzol (p-Phenylendiamin) und 1-Methyl-2,5-diaminobenzol (p-Toluylendiamin). Die Verwendung dieser Substanzen wird den farbtechnischen Wünschen der Anwender zwar weitgehend gerecht, es gibt jedoch immer noch Farbnuancen, die dadurch nicht voll erreicht bzw. noch intensiviert werden können.

Es wurde auch bereits vorgeschlagen, diese Lücke durch Verwendung alternativer Entwicklersubstanzen zu schließen. Dies ist in beschränktem Umfang möglich durch den Einsatz von 2-(2,5-Diaminophenyl)ethanol (vgl. EP-A 7537 und EP-B 400 330); jedoch müssen dann Abstriche in der Farbintensität anderer Nuancen hingenommen werden.
Eine weitere befriedigende Lösung dieses Problems wird auch durch den in der EP-A 615 743 beschriebenen Einsatz von 2-(2'-Hydroxyethylamino)-5-aminotoluol bzw. dessen wasserlöslichen Salzen als Entwicklersubstanzen in Haarfärbemitteln erreicht.

WO 96/09 807 A1 offenbart 2-hydroxy-1-ethanon Derivate, beispielweise Acetoin, Hydroxyaceton and Dihydroxyaceton enthaltende Haarfärbezusammensetzungen. Das Dokument beschreibt Verwendung von 2-Hydroxy-1-ethanon Derivate in Abwesenheit von Kuppler- und oxidierenden Substanzen.

Selbst dadurch bleiben jedoch noch farbtechnische Wünsche offen.

Auch Pyrazol und dessen Derivate wie 3,4-Diamino-5-hydroxypyrazol sind bereits als Bestandteil von Oxidations-Haarfärbemitteln vorgeschlagen worden.
Es war jedoch bisher nicht möglich, damit Färbungen in allen wünschenswerten Farbtönen und vor allem in der wünschenswerten Intensität herzustellen.

Die Erfindung geht daher von der Aufgabenstellung aus, ein Haarfärbemittel zu schaffen, das zur Herstellung einer großen Anzahl von Farbtönen geeignet ist und vor allem eine besonders intensive glänzende Färbung bewirkt.

Diese Aufgabe wird dadurch gelöst, daß ein solches Haarfärbemittel mindestens ein mit Peroxid reagierendes Oxidationsfarbstoff-System enthält, das aus einer Kombination aus mindestens einem Pyrazol bzw. dessen wasserlöslichen Salzen und/oder einem substituierten Triazol und mindestens eine Kupplersubstanz, beispielsweise ausgewählt aus 2-Amino-4-chlorphenol, 5-Amino-4-methoxy-2-methylphenol, 2-Aminophenol, 3-Aminophenol, 1-Methyl-2-hydroxy-4-aminobenzol, 3-N,N-Dimethylaminophenol, 2,6-Dihydroxy-3,5-dimethoxypyridin, 5-Amino-3-methylphenol, 6-Amino-3-methylphenol, 3-Amino-2-methylamino-6-methoxypyridin, 2-Amino-3-hydroxypyridin, 2-Dimethylamino-5-aminopyridin, 2,6-Diaminopyridin, 1,3-Diaminobenzol, 1-Amino-3-(2'-hydroxyethylamino)benzol, 1-Amino-3-[bis(2'-hydroxyethyl)amino]benzol, α-Naphthol, 4,6-Dichlorresorcin, 1,3-Diaminotoluol, 1-Hydroxynaphthalin, 4-Hydroxy-1,2-methylendioxybenzol, 1,5-Dihydroxynaphthalin, 1,6-Dihydroxynaphthalin, 1,7-Dihydroxynaphthalin, 2,7-Dihydroxynaphthalin, 1-Hydroxy-2-methylnaphthalin, 4-Hydroxy-1,2-methyldioxybenzol, 2,4-Diamino-3-chlorphenol und/oder 1-Methoxy-2-amino-4-(2'-hydroxyethylamino)benzol bzw. deren wasserlöslichen Salzen, und 0,01 bis 10, vorzugsweise 0,05 bis 5 Gew.-% Dihydroxyaceton, berechnet auf die Gesamtmenge des Mittels, in wäßriger Grundlage enthält.

Bevorzugte Pyrazole sind dabei Pyrazol selbst sowie die substituierten Pyrazole 1-Hydroxyethyl-4,5-diaminopyrazol, 3,4-Diamino-5-hydroxypyrazol, 3,5-Diaminopyrazol, 3,5-Diaminopyrazol-1-carboxamid, 3-Amino-5-hydroxypyrazol, 1-Phenyl-2-methylpyrazol, 1-Phenyl-3-methylpyrazol-5-on, 3,5-Dimethylpyrazol, 3,5-Dimethylpyrazol-1-methanol und/oder 3,5-Diamino-1,2,4-triazol bzw. deren wasserlösliche Salze.

Damit soll jedoch der Zusatz weiterer Entwickler- und Kupplersubstanzen keineswegs ausgeschlossen sein.
Bei Anwendung dieser Zusammensetzungen auf Basis einer üblichen Grundlage werden nach der Oxidation mit Peroxid sehr ausdrucksvolle, intensive, dauerhafte Haarfärbungen erhalten, die durch Zusatz entsprechender weiterer Entwickler- und Kupplersubstanzen noch zu anderen Farbnuancen variiert werden können.
Auch die zusätzliche Mitverwendung weiterer, an sich bekannter Entwicklersubstanzen ist möglich.
Als solche sind vorzugsweise 1,4-Diaminobenzol und substituierte p-Phenylendiamine, insbesondere 2,5-Diaminotoluol, 2-n-Propyl- bzw. 2-Ethyl-p-phenylendiamin, 2,6-Dimethyl-p-phenylendiamin, 2-(2,5-Diaminophenylen)ethanol, 1-Amino-4-bis-(2'-hydroxyethyl)aminobenzol, 2-(-2-Hydroxyethylamino)-5-aminotoluol, 4,4'-Diaminodiphenylamin, 4-Aminodiphenylamin, 2-Amino-5-N,N-diethylaminotoluol, 4-Amino-N-ethyl-N-isopropylanilin, 2-Chlor-p-phenylendiamin, 1β-Hydroxyethyl-2,5-diamino-4-methylbenzol, 2-Methoxy-p-phenylendiamin, N,N-Diethyl-p-phenylendiamin, 1-Amino-4-β-methoxyethylaminobenzol, 1-Dimethylamino-4-aminobenzol, 1-Hydroxy-2,5-diamino-4-methylbenzol, 1-Hydroxymethyl-2,5-diaminobenzol, 1,3-Dimethyl-2,5-diaminobenzol, 1,4-Diaminoisopropylbenzol, 1-Amino-4-β-hydroxypropylaminobenzol, 2-Aminophenol, 4-Aminophenole und deren Derivate, Tetraaminopyrimidine, Triaminohydroxypyrimidine, Diaminodihydroxypyrimidine, 5-Aminosalicylsäure und/oder 1,2,4-Triaminobenzol bzw. deren wasserlösliche Salze zu erwähnen.
Die Gesamtkonzentration der Entwicklersubstanzen liegt üblicherweise zwischen etwa 0,05 und 5 %, vorzugsweise 0,1 und 4 %, insbesondere 0,25 bis 0,5 % und 2,5 bis 3 % Gew.-% der Gesamtzusammensetzung des Haarfärbemittels (ohne Oxidationsmittel), wobei sich die Angaben jeweils auf den Anteil an freier Base beziehen.
Das bevorzugte Gewichtsverhältnis der genannten Entwicklersubstanzen zu den weiteren Entwickler- und Kupplersubstanzen liegt dabei zwischen etwa 1 : 8 bis 8 : 1, vorzugsweise etwa 1 : 5 bis 5 : 1, insbesondere 1 : 2 bis 2 : 1

Die Kupplersubstanz(en) als Reaktionspartner der Entwicklersubstanz(en) liegen in den erfindungsgemäßen Haarfärbemitteln etwa im gleichen molaren Anteil wie die Entwicklersubstanzen vor, d. h., also in Mengen von 0,01 bis 5,0 %, vorzugsweise 0,05 bis 4 %, insbesondere 0,1 bis 3 Gew.-% der Gesamtzusammensetzung (ohne Oxidationsmittel), wobei sich die Angaben jeweils auf den Anteil an freier Base beziehen.
Der Anteil an Dihydroxyaceton liegt vorzugsweise bei etwa 0,25 bis 2,5, insbesondere bei etwa 0,5 bis 2 Gew.-% des Farbmittels (ohne Oxidationsmittelzusammensetzung).
Die erfindungsgemäßen Zusammensetzungen können erwünschtenfalls auch sogenannte Nuanceure zur Feineinstellung des gewünschten Farbtones, insbesondere auch direktziehende Farbstoffe, enthalten.
Solche Nuanceure sind beispielsweise Nitrofarbstoffe wie 2-Amino-4,6-dinitrophenol, 2-Amino-4-nitrophenol, 2-Amino-6-chlor-4-nitrophenol, etc., vorzugsweise in Mengen von etwa 0,05 bis 2,5 %, insbesondere 0,1 bis 1 % Gew.-% der Farbzusammensetzung (ohne Oxidationsmittel).

Die erfindungsgemäßen Haarfärbemittel können die in solchen Mitteln üblichen Grund- und Zusatzstoffe, Konditioniermittel, etc. enthalten, die dem Fachmann aus dem Stand der Technik bekannt und beispielsweise in der Monographie von K. Schrader, "Grundlagen und Rezepturen der Kosmetika", 2. Aufl. (Hüthig Buch Verlag, Heidelberg, 1989), S. 782 bis 815, beschrieben sind. Sie können als Lösungen, Cremes, Gele oder auch in Form von Aerosol-Präparaten vorliegen; geeignete Trägermaterial-Zusammensetzungen sind aus dem Stand der Technik hinreichend bekannt.
Zur Applikation wird das erfindungsgemäße Oxidationsfarbstoff-Vorprodukt mit einem Oxidationsmittel vermischt. Bevorzugtes Oxidationsmittel ist Wasserstoffperoxid, beispielsweise in 2- bis 12-prozentiger Konzentration.
Es können jedoch auch andere Peroxide wie Harnstoffperoxid und Melaminperoxid eingesetzt werden.

Alternativ zur Peroxidoxidation kann auch eine Luftoxidation vorgenommen werden, beispielsweise, indem eine ein Oxidationsfarbstoffvorprodukt enthaltende Zusammensetzung als Aerosolschaum auf das Haar aufgebracht und dort für etwa 15 bis 30 Minuten einwirken gelassen wird.
Der pH-Wert des applikationsfertigen Haarfärbemittels, d. h. nach Vermischung mit Peroxid, kann sowohl im schwach sauren, d. h. einem Bereich von 5,5 bis 6,9, im neutralen als auch im alkalischen Bereich, d. h. zwischen pH 7,1 und 11 liegen.

Im folgenden werden verschiedene Ausführungsbeispiele zur Erläuterung der Erfindung gegeben.

| Grundlage | |
|---|---|
| Stearylalkohol | 8,0 (Gew.-%) |
| Kokosfettsäuremonoethanolamid | 4,5 |
| 1,2-Propandiolmono/distearat | 1,3 |
| Kokosfettalkoholpolyglykolether | 4,0 |
| Natriumlaurylsulfat | 1,0 |
| Ölsäure | 2,0 |
| 1,2-Propandiol | 1,5 |
| Na-EDTA | 0,5 |
| Natriumsulfit | 1,0 |
| Eiweißhydrolysat | 0,5 |
| Ascorbinsäure | 0,2 |
| Parfum | 0,4 |
| Ammoniak, 25%ig | 1,0 |
| Ammoniumchlorid | 0,5 |
| Panthenol | 0,8 |
| Wasser | ad 100,00 |

Die erfindungsgemäßen Oxidationsfarbstoff-Kombinationen wurden, unter entsprechender Verringerung des Wassergehalts, in diese Grundlage eingearbeitet.

Die Ausfärbungen erfolgten jeweils an Woll-Läppchen und Strähnen aus gebleichtem Menschenhaar, durch Aufbringen einer 1:1-Mischung aus Farbstoff-Vorprodukt und 6%iger Wasserstoffperoxid-Lösung (pH-Wert der Mischung: 9,8) und zwanzigminütiger Einwirkung bei Zimmertemperatur, folgendem Auswaschen und Trocknen.

Es wurden die folgenden Färbungen erzielt:

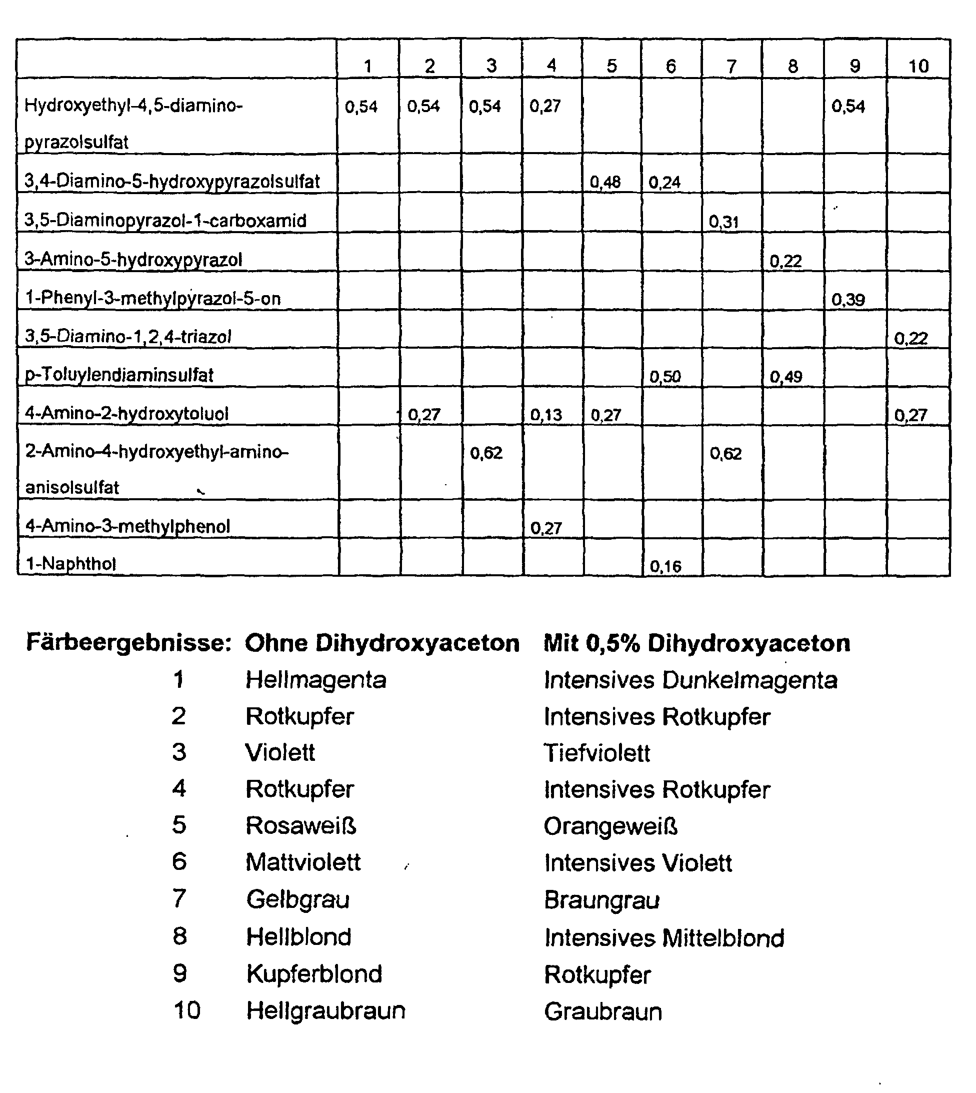

In die beschriebene Grundlage wurde die folgende Oxidationsfarbstoffmischung jeweils mit und ohne 0,5 Gew.-% Dihydroxyaceton eingebracht und der pH-Wert so eingestellt, daß beim Vermischen mit 2%-iger wäßriger H₂O₂-Lösung im Gewichtsverhältnis 1:1 ein pH-Wert der applikationsfertigen Mischung von 6,8 erreicht wurde.
Die Mischung wurde wiederum jeweils auf Woll-Läppchen und Strähnen aus gebleichtem Menschenhaar aufgebracht, nach 15-minütiger Einwirkung ausgewaschen und getrocknet und die Färbung bewertet.

Es wurde folgendes Ergebnis erzielt:

### Zusammensetzung

| Zusammensetzung | | |
|---|---|---|
| | Nr. 1 | Nr. 1a |
| 1-Hydroxyethyl-4,5-diaminopyrazolsulfat | 0,54 % | 0,54 % |
| 4-Amino-2-hydroxytoluol | 0,27 % | 0,27 % |
| Dihydroxyaceton | 0,50 % | ― |
| Färbung | Intensives Rotkupfer | Rotkupfer |

Die erfindungsgemäße Zusammensetzung Nr. 1 erzielt eine glänzende, intensive Ausfärbung, die derjenigen der Zusammensetzung 1a deutlich überlegen war.

## Patentansprüche

1. Mittel zum Färben von menschlichen Haaren, enthaltend mindestens ein Oxidationsfarbstoffvorprodukt, ausgewählt aus Pyrazol und seinen Derivaten bzw. deren wasserlöslichen Salzen und/oder einem substituierten Triazol, und mindestens eine Kupplersubstanz, ausgewählt aus, 2-Amino-4-chlorphenol, 5-Amino-4-methoxy-2-methylphenol, 2-Aminophenol, 3-Aminophenol, 1-Methyl-2-hydroxy-4-aminobenzol, 3-N,N-Dimethylaminophenol, 2,6-Dihydroxy-3,5-dimethoxypyridin, 5-Amino-3-methylphenol, 6-Amino-3-methylphenol, 3-Amino-2-methylamino-6-methoxypyridin, 2-Amino-3-hydroxypyridin, 5-Amino-2-methoxyphenol, 2-Dimethylamino-5-aminopyridin, 2,6-Diaminopyridin, 1,3-Diaminobenzol, 1-Amino-3-(2'-hydroxyethylamino)benzol, 1-Amino-3-[bis(2'hydroxyethyl)amino]benzol, α-Naphthol, 4,6-Dichlorresorcin, 1,3-Diaminotoluol, 1-Hydroxynaphthalin, 4-Hydroxy-1,2-methylendioxybenzol, 1,5-Dihydroxynaphthalin, 1,6-Dihydroxynaphthalin, 1,7-Dihydroxynaphthalin, 2,7-Dihydroxynaphthalin, 1-Hydroxy-2-methylnaphthalin, 2,4-Diamino-3-chlorphenol und/oder 1-Methoxy-2-amino-4-(2'-hydroxyethylamino)benzol bzw. deren wasserlöslichen Salzen in wäßriger Grundlage, **gekennzeichnet durch** einen Gehalt an 0,01 bis 10 Gew.-% Dihydroxyaceton, berechnet auf die Gesamtzusammensetzung des Mittels.

2. Mittel nach Anspruch 1, enthaltend als Pyrazol- bzw. Triazolderivat mindestens eine Verbindung ausgewählt aus 1-Hydroxyethyl-4,5-diaminopyrazol, 3,4-Diamino-5-hydroxypyrazol, 3,5-Diaminopyrazol, 3,5-Diaminopyrazol-1-carboxamid, 3-Amino-5-hydroxypyrazol, 1-Phenyl-2-methylpyrazol, 1-Phenyl-3-methylpyrazol-5-on, 3,5-Dimethylpyrazol, 3,5-Dimethylpyrazol-1-methanol und/oder 3,5-Diamino-1,2,4-triazol bzw. deren wasserlösliche Salze.

3. Mittel nach den Ansprüchen 1 und 2, enthaltend 0,05 bis 5 Gew.-% Dihydroxyaceton, bezogen auf die Gesamtzusammensetzung.

4. Verwendung von Dihydroxyaceton in Haarfärbemitteln auf Basis mindestens eines Oxidationsfarbstoffvorprodukts, ausgewählt aus Pyrazol und seinen Derivaten und/oder substituierten Triazolen bzw. deren wasserlöslichen Salzen und und mindestens eine Kupplersubstanz, ausgewählt aus, 2-Amino-4-chlorphenol, 5-Amino-4-methoxy-2-methylphenol, 2-Aminophenol, 3-Aminophenol, 1-Methyl-2-hydroxy-4-aminobenzol, 3-N,N-Dimethylaminophenol, 2,6-Dihy-droxy-3,5-dimethoxypyridin, 5-Amino-3-methylphenol, 6-Amino-3-methylphenol, 3-Amino-2-methylamino-6-methoxypyridin, 2-Amino-3-hydroxypyridin, 5-Amino-2-methoxyphenol, 2-Dimethylamino-5-aminopyridin, 2,6-Diaminopyridin, 1,3-Diaminobenzol, 1-Amino-3-(2'-hydroxyethylamino)benzol, 1-Amino-3-[bis(2'hydroxyethyl)amino]benzol, α-Naphthol, 4,6-Dichlorresorcin, 1,3-Diaminotoluol, 1-Hydroxynaphthalin, 4-Hydroxy-1,2-methylendioxybenzol, 1,5-Dihydroxynaphthalin, 1,6-Dihydroxynaphthalin, 1,7-Dihydroxynaphthalin, 2,7-Dihydroxynaphthalin, 1-Hydroxy-2-methylnaphthalin, 2,4-Diamino-3-chlorphenol und/oder 1-Methoxy-2-amino-4-(2'-hydroxyethylamino)benzol bzw. deren wasserlöslichen Salzen.

## Claims

1. Composition for the dyeing of human hair, comprising at least one oxidation dyestuff precursor, selected from pyrazole and the derivatives thereof or the water-soluble salts thereof and/or a substituted triazole and at least one coupling substance selected from 2-amino-4-chlorophenol, 5-amino-4-methoxy-2-methylphenol, 2-aminophenol, 3-amino-phenol, 1-methyl-2-hydroxy-4-aminobenzene, 3-N,N-dimethyl aminophenol, 2,6-dihydroxy-3,5-dimethoxypyridine, 5-amino-3-methylphenol, 6-amino-3-methylphenol, 3-amino-2-methylamino-6-methoxypyridine, 2-amino-3-hydroxy-pyridine, 2-dimethylamino-5-aminopyridine, 2.6-diaminopyridine, 1,3-diaminobenzene, 1-amino-3-(2'-hy-droxyethylamino)benzene, 1-amino-3-[bis(2'-hydroxy-ethyl) amino]benzene, α-naphthol, 4,6-dichlororesorcinol, 1,3-diaminotoluene, 1-hydroxy naphthalene, 4-hydroxy-1,2-methylenedioxy benzene, 1,5-dihydroxy naphthalene, 1,6-dihydroxy naphthalene, 1,7-dihydroxy naphthalene, 2,7-dihydroxy naphthalene, 1-hydroxy-2-methyl naphthalene, 4-hydroxy-1,2-methyldioxy benzene, 2,4-diamino-3-chlorophenol and/or 1-methoxy-2-amino-4-(2'-hydroxyethyl amino)benzene or the water-soluble salts thereof in aqueous carrier, **characterized in** a content of 0.01 % to 10 % by weight of dihydroxy acetone, calculated to the total composition.

2. Composition according to claim 1, comprising as pyrazole or triazole derivative at least one compound selected from 1-hydroxyethyl-4,5-diaminopyrazole, 3,4-diamino-5-hydroxypyrazole, 3,5-diaminopyrazole, 3,5-diaminopyrazole-1-carboxamide, 3-amino-5-hydroxypyrazole, 1-phenyl-2-methylpyrazole, 1-phenyl-3-methylpyrazole-5-one, 3,5-dimethylpyrazole, 3,5-dimethylpyrazole-1-methanol and/or 3,5-diamino-1,2,4-triazole or the water-soluble salts thereof.

3. Composition according to claim 1 and 2, **characterized in that** it comprises 0.05 % to 5 % by weight of dihydroxy acetone, calculated to the total composition.

4. Use of dihydroxy acetone in hair dyeing compositions on the basis of at least one oxidation dyestuff precursor, selected from pyrazole and the derivatives thereof and/or substituted triazoles or the water-soluble salts thereof and at least one coupling substance selected from 2-amino-4-chlorophenol, 5-amino-4-methoxy-2-methyl-phenol, 2-aminophenol, 3-amino-phenol, 1-methyl-2-hydroxy-4-aminobenzene, 3-N,N-dimethyl aminophenol, 2,6-dihydroxy-3,5-dimethoxypyridine, 5-amino-3-methylphenol, 6-amino-3-methylphenol, 3-amino-2-methylamino-6-methoxy-pyridine, 2-amino-3-hydroxy-pyridine, 2-dimethylamino-5-aminopyridine, 2.6-diaminopyridine, 1.3-diaminobenzene, 1-amino-3-(2'-hydroxyethyl-amino)benzene, 1-amino-3-[bis(2'-hydroxy-ethyl) amino]benzene, α-naphthol, 4,6-dichlororesorcinol, 1,3-diaminotoluene, 1-hydroxy naphthalene, 4-hydroxy-1,2-methylenedioxy benzene, 1,5-dihydroxy naphthalene, 1,6-dihydroxy naphthalene, 1,7-dihydroxy naphthalene, 2,7-dihydroxy naphthalene, 1-hydroxy-2-methyl naphthalene, 4-hydroxy-1,2-methyldioxy benzene, 2,4-diamino-3-chlorophenol and/or 1-methoxy-2-amino-4-(2'-hydroxyethyl amino)benzene or the water-soluble salts thereof.

## Revendications

1. Agent de coloration des cheveux humains, qui contient au moins un précurseur de colorant par oxydation sélectionné parmi le pyrazole et ses dérivés ou sels solubles dans l'eau et/ou un triazole substitué, et au moins une substance de couplage sélectionnée parmi le 2-amino-4-chlorophénol, le 5-amino-4-méthoxy-2-méthylphénol, le 2-aminophénol, le 3-aminophénol, le 1-méthyl-2-hydroxy-4-aminobenzène, le 3-N,N-diméthylaminophénol, la 2,6-dihydroxy-3,5-diméthoxypyridine, le 5-amino-3-méthylphénol, le 6-amino-3-méthylphénol, la 3-amino-2-méthylamino-6-méthoxypyridine, la 2-amino-3-hydroxypyridine, le 5-amino-2-méthoxyphénol, la 2-diméthylamino-5-aminopyridine, la 2,6-diaminopyridine, le 1,3-diaminobenzène, le 1-amino-3-(2'-hydroxyéthylamino)benzène, le 1-amino-3-[bis(2'-hydroxyéthyl)amino]benzène, l'α-naphtène, la 4,6-dichlororésorcine, le 1,3-diaminotoluène, la 1-hydroxynaphtaline, le 4-hydroxy-1,2-méthylènedioxybenzène, la 1,5-dihydroxynaphtaline, la 1,6-dihydroxynaphtaline, la 1,7-dihydroxynaphtaline, la 2,7-dihydroxynaphtaline, la 1-hydroxy-2-méthylnaphtaline, le 2,4-diamino-3-chlorophénol et/ou le 1-méthoxy-2-amino-4-(2'-hydroxyéthylamino)benzène et leurs sels solubles dans l'eau, à base aqueuse, **caractérisé par** une teneur de 0,01 à 10 % en poids en dihydroxyacétone calculée par rapport à la composition totale de l'agent.

2. Agent selon la revendication 1, qui contient comme pyrazole ou dérivé de triazole au moins un composé sélectionné parmi le 1-hydroxyéthyl-4,5-diaminopyrazole, le 3,4-diamino-5-hydroxypyrazole, le 3,5-diaminopyrazole, le 3,5-diaminopyrazole-1-carboxamide, le 3-amino-5-hydroxypyrazole, le 1-phényl-2-méthylpyrazole, la 1-phényl-3-méthylpyrazole-5-one, le 3,5-diméthylpyrazole, le 3,5-diméthylpyrazole-1-méthanol et/ou le 3,5-diamino-1,2,4-triazole et leurs sels solubles dans l'eau.

3. Agent selon les revendications 1 et 2, qui contient de 0,05 à 5 % en poids de dihydroxyacétone par rapport à la composition totale.

4. Utilisation de dihydroxyacétone dans des agents de coloration des cheveux à base d'au moins un précurseur de colorant par oxydation sélectionné parmi le pyrazole et ses dérivés et/ou les triazoles substitués et leurs sels solubles dans l'eau et d'au moins une substance de couplage sélectionnée parmi le 2-amino-4-chlorophénol, le 5-amino-4-méthoxy-2-méthylphénol, le 2-aminophénol, le 3-aminophénol, le 1-méthyl-2-hydroxy-4-aminobenzène, le 3-N,N-diméthylaminophénol, la 2,6-dihydroxy-3,5-diméthoxypyridine, le 5-amino-3-méthylphénol, le 6-amino-3-méthylphénol, la 3-amino-2-méthylamino-6-méthoxypyridine, la 2-amino-3-hydroxypyridine, le 5-amino-2-méthoxyphénol, la 2-diméthylamino-5-aminopyridine, la 2,6-diaminopyridine, le 1,3-diaminobenzène, le 1-amino-3-(2'-hydroxyéthylamino)benzène, le 1-amino-3-[bis(2'-hydroxyéthyl)amino]benzène, l'α-naphtène, la 4,6-dichlororésorcine, le 1,3-diaminotoluène, la 1-hydroxynaphtaline, le 4-hydroxy-1,2-méthylènedioxybenzène, la 1,5-dihydroxynaphtaline, la 1,6-dihydroxynaphtaline, la 1,7-dihydroxynaphtaline, la 2,7-dihydroxynaphtaline, la 1-hydroxy-2-méthylnaphtaline, le 2,4-diamino-3-chlorophénol et/ou le 1-méthoxy-2-amino-4-(2'-hydroxyéthylamino)benzène et leurs sels solubles dans l'eau.
